**Europäisches Patentamt**

**European Patent Office**

**Office européen des brevets**

(19)

(11) Veröffentlichungsnummer : **0 343 575 B1**

(12) # EUROPÄISCHE PATENTSCHRIFT

(45) Veröffentlichungstag der Patentschrift :
**26.08.92 Patentblatt 92/35**

(51) Int. Cl.⁵ : **A61K 9/48,** A61K 31/80

(21) Anmeldenummer : **89109215.7**

(22) Anmeldetag : **23.05.89**

(54) **Weichgelatinekapsel.**

(30) Priorität : **24.05.88 DE 3818022**

(43) Veröffentlichungstag der Anmeldung :
**29.11.89 Patentblatt 89/48**

(45) Bekanntmachung des Hinweises auf die
Patenterteilung :
**26.08.92 Patentblatt 92/35**

(84) Benannte Vertragsstaaten :
**AT BE CH DE ES FR GB GR IT LI LU NL SE**

(56) Entgegenhaltungen :
**FR-A- 2 077 790**
**FR-A- 2 372 635**
**FR-A- 2 383 661**
**FR-A- 2 390 953**

(73) Patentinhaber : **Stephan, Günter**
**Happoldstrasse 47**
**W-7000 Stuttgart 30 (DE)**

(72) Erfinder : **Stephan, Günter**
**Happoldstrasse 47**
**W-7000 Stuttgart 30 (DE)**
Erfinder : **Stephan, Dieter, Dr. Dipl.-Chem.**
**Gehrenwaldstrasse 35 A**
**W-7000 Stuttgart 60 (DE)**
Erfinder : **Honerlagen, Hans**
**Binsenmatt 2**
**CH-6314 Unterägri (CH)**
Erfinder : **Mitschka, Jochen**
**Strünkerhof 13a**
**W-5304 Much (DE)**

(74) Vertreter : **Patentanwälte Wenzel & Kalkoff**
**Grubes Allee 26 Postfach 730466**
**W-2000 Hamburg 73 (DE)**

## Beschreibung

Die Erfindung betrifft eine Weichgelatinekapsel, enthaltend einen mit Öl als Trägersubstanz gemischten therapeutischen Wirkstoff, insbesondere für die Therapie des Magen-Darm-Bereiches. Die Mischung kann dabei als Emulsion, Suspension, Dispersion, Lösung oder Gemenge vorliegen.

Weichgelatinekapseln eignen sich bekanntermaßen als günstige Verabreichungsform von Arzneimitteln verschiedenster Art, da sie in herstellungstechnisch einfacher Weise Wirkstoffe und Träger in jeweils erforderlichem Verhältnis aufnehmen und rasch eine hohe Wirksamkeit entfalten können. Als Beispiel hierfür sei die durch die EP-A1 178 436 bekannt gewordene Darreichungsform von gelöstem Ibuprofen mit Hilfe von Weichgelatinekapseln genannt, wobei dieses Schmerzmittel nach Verabreichung besonders rasch resorbiert wird und man damit einen schnellen und zuverlässigen Wirkungseintritt erreicht.

Bei der Herstellung von Weichgelatinekapseln ist es heute üblich, daß die zu verkapselnden Wirkstoffe (wirksamen Substanzen) in eine über Pumpen dosierbare Form gebracht werden. Als Lösungsmittel hierfür scheiden Wasser und niedere Alkohole aus, da diese in der Kapsel nicht stabil sind. Bevorzugte Lösungsmittel sind Pflanzenöle, Neutralöle und Polyethylenglykole. Substanzen, die nicht löslich sind, müssen in eine möglichst stabile Emulsionsform gebracht werden. Als Emulgatoren werden Polyethylenglykole, u.a. Lecithine, hydrierte Pflanzenöle und Bienenwachs verwendet.

Der Erfindung liegt die Aufgabe zugrunde, den Wirkstoff, insbesondere eine pulverige Substanz, in eine direkt mit Siliconöl oder-mischung zu verkapselnde Form zu bringen.

Diese Aufgabe wird nach der Erfindung dadurch gelöst, daß die Trägersubstanz ein Siliconöl oder eine Siliconölmischung ist und diese Trägestoff der einzige Betandteil neben dem thrapendischen Wirkstoff ist.

Gegenüber den herkömmlichen Weichgelatinekapseln bzw. deren Herstellung lassen sich durch die erfindungsgemäße Verwendung der auf zahlreichen Gebieten wie in der Pharmazie, Medizin, Kosmetik, und im Lebensmittelbereich im Einsatz befindlichen Siliconöle/-Mischungen erhebliche Vorteile erzielen. So ist es einmal möglich, eine stabile Wirkstoff-Trägersubstanz-Mischung, insbesondere Siliconöl-Emulsion ohne Zuhilfenahme weiterer Zusatzmittel, insbesondere Emulgatoren, zu erzeugen, wobei je nach der zu verarbeitenden Wirksubstanz die Viskosität des Siliconöls eingestellt wird. Hierfür steht ein breiter Auswahlbereich zur Verfügung, da Siliconöle heute in Viskositäten von 1 bis 300 000 mm²/s in Pharmaqualitäten zur Verfügung stehen. Dabei ist festgestellt worden, daß die Viskosität der erfindungsgemäßen Trägersubstanz mit Zunahme der Wirkstoffmenge abnehmen und entsprechend bei einer geringen Wirkstoffmenge relativ hoch gewählt werden sollte. Ein wesentlicher Vorteil der Erfindung ist darin zu sehen, daß Siliconöle selbst als Wirkstoffe einen therapeutischen Wert in der Magen-Darm-Therapie haben, beispielsweise bei Blähungen (Roemheld-Syndrom) . Da bekanntermaßen bei verschiedenen Magen-Darm-Präparaten, insbesondere bei Abführmitteln auf pflanzlicher Basis, als Nebenwirkungen Blähungen und Unwohlsein auftreten, ist der therapeutische Effekt von Siliconölen gegen solche Symptome besonders wertvoll. Das Herstellungsverfahren läßt sich durch den Wegfall zusätzlicher Verbesserer maßgeblich vereinfachen, indem vorgeschaltete Mischvorgänge entfallen und insgesamt nur zwei Komponenten in Emulsion gebracht zu werden brauchen.

Somit ergibt sich insbesondere dann, wenn als Wirkstoff ein Pflanzenextrakt, beispielsweise ein Extrakt Sennae sicc. eingesetzt wird, eine doppelte Funktion des Emulgators, der neben seiner eigentlichen Aufgabe unangenehme Nebenwirkungen verhindert und unterdrückt. Für solche Pflanzenextrakte hat sich als besonders zweckmäßig eine Siliconöl-Viskosität von 300 mm²/s entsprechend obigen Maßgaben für die Viskositätswahl herausgestellt. Dabei macht man sich als Vorteil zunutze, daß Sennoside, d.h. die Wirkstoffe des Sennae Extraktes, im wässrigen Milieu unstabil sind. Aus diesem Grunde empfiehlt es sich, einen wasserfreien Hilfsstoff einzusetzen. Damit ist ein hydrophober Träger wie z.B. Siliconöl als besonders günstig in der Kombination mit Pflanzenextrakten dieser Art anzusehen. Als weitere Wirkstoffe kommen aber auch andere Pflanzenextrakte und Substanzen in Betracht, die bei Magen-Darm-Beschwerden Verwendung finden, vor allem Extr. Chelidonii, Extr. Absinthii, Extr. Rhei, Extr. Cynarae, Extr. Cascarae, Extr. Frangulae, Extr. Aloe, Extr. Uvae Ursi oder auch Mischungen der pflanzlichen Wirkstoffe.

Neben den genannten Pflanzenextrakten können als Wirkstoffe auch chemotherapeutische Stoffe eingesetzt werden. Bevorzugte chemotherapeutische Stoffe sind Bisacodyl und Natriumspicosulfat. Dabei kann die in der Weichgelatinekapsel aufgenommene Mischung vorteilhaft eine Suspension enthaltend ca. 98 Gew.% Siliconöl einer Viskosität von 12 500 mm²/s und ca. 2 Gew.% Bisacodyl bzw. Natriumpicosulfat sein.

Aus Herstellungs- und Kostengründen, aber auch im Hinblick auf die Verabreichungserfordernisse, ist es ganz allgemein sinnvoll und zweckmäßig, wenn die Menge des Wirkstoffes 0,2 bis 75 Gew.% und die der Trägersubstanz Siliconöl/Siliconölmischung 99,8 bis 25 Gew.% beträgt.

Weitere Vorteile und Zweckmäßigkeiten der Erfindung gehen aus den im folgenden erörterten Ausführungsbeispielen hervor.

Beispiel 1

Es werden 43,3885 g Silikon einer Viskosität von 12 500 mm²/s (Hersteller: Wacker-Chemie GmbH) mit 0,8870 g Bisacodyl (Hersteller: Böhringer, Ingelheim) unter 30minütigem Rühren bei Raumtemperatur und einer Rührgeschwindigkeit von 500 U/min gemischt. Die Mischung wird über einen Walzenstuhl gegeben, und zwar für ca. 15 min. Die erhaltene Suspension ist nach 2 Tagen noch völlig stabil und kann verkapselt werden. In einer 5 oval Kapsel mit einem Füllgewicht von 250 mg ergibt dies einen Gehalt von 5 mg des chemotherapeutischen Wirkstoffs.

Beispiel 2

Für eine Mischung aus

| | |
|---|---|
| 5 mg | Natriumpicosulfat (Profarmaco CH.B 170104) und |
| 215 mg | Siliconöl AK 12500 (Wacker Chemie; Viskosität 12500 mm²/s; CH.B 1024 OH) zur Herstellung einer |
| 4-oval | Kapsel mit 220 mg Füllgewicht wird eine Einwaage von |
| 27,742 g | Siliconöl und |
| 0,643 g | (theoretische Menge 0,645) Na-picosulfat gerührt, im Laborwalzenstuhl homogenisiert und im Exsikkator entlüftet. Es ergibt sich eine stabile, gut zu verkapseldne Suspension. |

Beispiel 3

Für eine Mischung aus

| | |
|---|---|
| 250 mg | Extr. Sennae (FLA 85.414) und |
| 200 mg | Siliconöl 70047V100 (Rhône Poulenc; Viskosität 100 mm²/s) zur Herstellung einer |
| 7,5 oval | Kapsel mit 450 mg Füllgewicht wird eine Einwaage von |
| 43,087 g | (theoretische Menge 43.0725) Extr. Sennae und |
| 34,458 g | Siliconöl gerührt, im Laborwalzenstuhl homogenisiert und im Exsikkator entlüftet. Er ergibt sich eine stabile, gut zu verkapselnde Suspension. |

Beispiel 4

Für eine Mischung aus

| | |
|---|---|
| 250 mg | Extr. Chelidonii 3,5 % (entsprechend 1,5 g Droge) und |
| 200 mg | Siliconöl 70047V100 (Rhône Poulenc; Viskosität 100 mm²/s) zur Herstellung einer |
| 7,5 oval | Kapsel mit 450 mg Füllgewicht wird eine Einwaage von |
| 40,789 g | Siliconöl und |
| 50,994 g | (theoretische Menge 50,986) Chelidonii gerührt, im Laborwalzenstuhl homogenisiert und im Exsikkator entlüftet. Es ergibt sich eine stabile, gut zu verkapselnde Suspension. |

Beispiel 5

Für eine Mischung aus

| | |
|---|---|
| 250 mg | Extr. Absinthii sicc. (6:1 30 % CH.B 0021 ECS) und |
| 300 mg | Siliconöl 70047V1000 (Rhône Poulenc; Viskosität 1000 mm²/s) zur Herstellung einer |
| 10 oval | Kapsel mit 550 mg Füllgewicht wird eine Einwaage von |
| 35,742 g | Siliconöl und |
| 29,600 g | (theoretische Menge 29,786) Extr. Absinthii gerührt, im Laborwalzenstuhl homogenisiert und im Exsikkator entlüftet. Es ergibt sich eine stabile, gut zu verkapselnde Suspension. |

Beispiel 6

Für eine Michung aus

| | |
|---|---|
| 300 mg | Extr. Rhei sicc., DAB 7 (10 % HAD CH.B 3983 ECS) und |
| 200 mg | Siliconöl 70047V5000 (Rhône Poulenc; Viskosität 5000 mm²/s) zur Herstellung einer |
| 10 oval | Kapsel mit 500 mg Füllgewicht wird eine Einwaage von |
| 37,960 g | Siliconöl und |
| 45,514 g | (theoretische Menge 45,552) Extr. Rhei gerührt, im Laborwalzenstuhl homogenisiert und im Ex- |

sikkator entlüftet. Es ergibt sich eine stabile, gut zu verkapselnde Suspension.

Beispiel 7

Für eine Mischsung aus

| | |
|---|---|
| 500 mg | Extr. Cynarae (FLA 85.437; Fa. Flachsmann) und |
| 500 mg | Siliconöl 70047V100 (Rhône Poulenc; Viskosität 100 mm²/s) zur Herstellung einer |
| 20 oblong | Kapsel mit 1000 mg Füllgewicht wird eine Einwaage von |
| 50,972 g | Siliconöl und |
| 50,972 g | (theoretische Menge 50,972) Extr. Cynarae gerührt, im Laborwalzenstuhl homogenisiert und im Exsikktator entlüftet. Es ergibt sich eine stabile, gut zu verkapselnde Suspension. |

Beispiel 8

Für eine Mischung aus

| | |
|---|---|
| 100 mg | Extr. Aloe DAB 9 (REI 19-21 % HAD; Reisholz GmbH) und |
| 100 mg | Siliconöl 70047V1000 (Rhône Poulenc; Viskosität 1000 mm²/s) zur Herstelung einer |
| 4 oval | Kapsel mit 200 mg Füllgewicht wird eine Einwaage von |
| 9,110 g | Extr. Aloe und |
| 9,183 g | (theoretische Menge 9,110) Siliconöl gerührt, im Laborwalzenstuhl homogenisiert und im Exsikkator entlüftet. Es ergibt sich eine stabile, gut zu verkapselnde Suspension. |

Beispiel 9

Für eine Mischung aus

| | |
|---|---|
| 500 mg | Extr. Uvae Ursi (Flachsmann CH.B 7D514) und |
| 400 mg | Siliconöl 70047V100 (Rhône Poulenc; Viskosität 100 mm²/s) zur Herstellung einer |
| 16 oblong | Kapsel mit 900 mg Füllgewicht wird eine Einwaage von |
| 37,171 g | Siliconöl und |
| 46,430 g | (theoretische Menge 46,464) Extr. Uvae Ursi gerührt, im Laborwalzenstuhl homogenisiert und im Exsikkator entlüftet. Es ergibt sich eine stabile, gut zu verkapselnde Suspension. |

**Patentansprüche**

1.  Weichgelatinekapsel, enthaltend einen mit Öl als Trägersubstanz gemischten therapeutischen Wirkstoff, insbesondere für die Therapie des Magen-Darm-Bereiches, **dadurch gekennzeichnet,** daß die Trägersubstanz ein Siliconöl oder eine Siliconölmischung ist und die Trägersubstanz der einzige Betandteil neben dem therapeutischen Wirkstoff ist.

2.  Weichgelatinekapsel nach Anspruch 1, **dadurch gekennzeichnet,** daß der Wirkstoff als pulverige Substanz in der Trägersubstanz Siliconöl bzw. Siliconölmischung emulgiert ist.

3.  Weichgelatinekapsel nach Anspruch 1 oder 2, **dadurch gekennzeichnet,** daß die Trägersubstanz eine Viskosität zwischen 1 und 200 000 mm²/s aufweist.

4.  Weichgelatinekapsel nach Anspruch 3, **dadurch gekennzeichnet,** daß der Trägersubstanz ein Siliconöl einer Viskosität von ungefähr 300 mm²/s ist.

5.  Weichgelatinekapsel nach einem der Ansprüche 1 bis 4, **dadurch gekennzeichnet,** daß die Menge des Wirkstoffes 0,2 bis 75 Gew.% und die der Trägersubstanz Siliconöl/Siliconölmischung 99,8 bis 25 Gew.% beträgt.

6.  Weichgelatinekapsel nach einem der Ansprüche 1 bis 5, **dadurch gekennzeichnet,** daß der Wirkstoff ein Pflanzenextrakt ist.

7.  Weichgelatinekapsel nach Anspruch 6, **dadurch gekennzeichnet,** daß der Wirkstoff ein Extrakt Sennae sicc., Extr. Chelidonii, Extr. Absinthii, Extr. Rhei, Extr. Cynarae, Extr. Cascarae, Extr. Frangulae, Extr. Aloe oder Extr. Uvae Ursi, oder eine Mischung derselben ist.

8. Weichgelatinekapsel nach einem der Ansprüche 1 bis 5, **dadurch gekennzeichnet,** daß der Wirkstoff ein chemotherapeutisches Mittel ist.

9. Weichgelatinekapsel nach Anspruch 8, **dadurch gekennzeichnet,** daß der Wirkstoff Bisacodyl ist.

10. Weichgelatinekapsel nach Anspruch 9, **dadurch gekennzeichnet,** daß die Mischung eine Suspension enthaltend ca. 98 Gew% Siliconöl einer Viskosität von 12 500 mm$^2$/s und ca. 2 Gew% Bisacodyl ist.

## Claims

1. A soft gelatine capsule, containing a therapeutic active material mixed with oil as the carrier substance, in particular for treatment of the stomach-intestinal region, characterised in that the carrier substance is a silicone oil or a silicone oil mixture and the carrier substance is the sole constituent, besides the therapeutic active material.

2. A soft gelatine capsule according to Claim 1, characterised in that the active material is emulsified as a powdered substance in the silicone oil or silicone oil mixture carrier substance.

3. A soft gelatine capsule according to Claim 1 or 2, characterised in that the carrier substance has a viscosity of between 1 and 200,000 mm$^2$/s.

4. A soft gelatine capsule according to Claim 3, characterised in that the carrier substance is a silicone oil having a viscosity of approximately 300 mm$^2$/s.

5. A soft gelatine capsule according to any one of Claims 1 to 4, characterised in that the quantity of active material is 0.2 to 75 % by weight and the quantity of the silicone oil/silicone oil mixture carrier substance is 99.8 to 25 % by weight.

6. A soft gelatine capsule according to any one of Claims 1 to 5, characterised in that the active material is a plant extract.

7. A soft gelatine capsule according to Claim 6, characterised in that the active material is a sennae sicc. extract, chelidonii extract, absinthii extract, rhei extract, cynarae extract, cascarae extract, frangulae extract, aloe extract or uvae ursi extract, or a mixture thereof.

8. A soft gelatine capsule according to any one of Claims 1 to 5, characterised in that the active material is a chemotherapeutic agent.

9. A soft gelatine capsule according to Claim 8, characterised in that the active material is bisacodyl.

10. A soft gelatine capsule according to Claim 9, characterised in that the mixture is a suspension containing approximately 98 % silicone oil having a viscosity of 12,500 mm$^2$/s and approximately 2 % by weight bisacodyl.

## Revendications

1. Capsule en gélatine molle renfermant un agent actif thérapeutique mélangé avec de l'huile comme substance porteuse, en particulier pour le traitement de la sphère gastro-intestinale caractérisée en ce que la substance porteuse est une huile de silicone ou un mélange d'huiles de silicone et la substance porteuse est le composant unique voisin de l'agent actif thérapeutique.

2. Capsule selon la revendication 1 caractérisée en ce que l'agent actif est une substance pulvérulente présente en émulsion dans la substance porteuse constituée par l'huile de silicone ou le mélange d'huiles de silicone.

3. Capsule selon l'une des revendications 1 ou 2 caractérisée en ce que la substance porteuse a une viscosité comprise entre 1 et 200 000 mm2/s.

4. Capsule selon la revendication 3 caractérisée en ce que la substance porteuse est une huile de silicone d'une viscosité d'environ 300 mm2/s.

5. Capsule selon l'une quelconque des revendications 1 à 4 caractérisée en ce que la quantité d'agent actif est de 0,2 à 75 % en poids et la substance porteuse, huile de silicone/mélange d'huiles de silicone est de 99,8 à 25 % en poids.

6. Capsule selon l'une des revendications 1 à 5 caractérisée en ce que l'agent actif est un extrait de plantes.

7. Capsule selon la revendication 6 caractérisée en ce que l'agent actif est un des extraits suivants de Sennae sicc, Chelidonii, Absinthii, Rhei, Cynarae, Cascarae, Frangulae, Aloe ou de Uvae Usi ou un mélange de ces extraits.

8. Capsule selon l'une des revendications 1 à 5 caractérisée en ce que l'agent actif est un moyen chimio thérapeutique.

9. Capsule selon la revendication 8 caractérisée en ce que l'agent actif est du Bisacodyl.

10. Capsule selon la revendication 9 caractérisée en ce que le mélange est une suspension contenant environ 98 % en poids d'huile de silicone d'une viscosité de 12 500 mm2/s et environ 2 % en poids de Bisacodyl.